(19) Europäisches Patentamt
European Patent Office
Office européen des brevets

(11) **EP 3 272 291 B1**

(12) **EUROPEAN PATENT SPECIFICATION**

(45) Date of publication and mention
of the grant of the patent:
**27.01.2021 Bulletin 2021/04**

(21) Application number: **15885653.4**

(22) Date of filing: **24.07.2015**

(51) Int Cl.:
*A61B 8/00* (2006.01)     *A61B 8/13* (2006.01)
*A61B 8/08* (2006.01)     *G06T 5/00* (2006.01)
*G06T 5/50* (2006.01)

(86) International application number:
**PCT/KR2015/007737**

(87) International publication number:
**WO 2016/148351 (22.09.2016 Gazette 2016/38)**

(54) **DEVICE AND METHOD FOR RECONSTRUCTING MEDICAL IMAGE**

VORRICHTUNG UND VERFAHREN ZUR REKONSTRUKTION EINES MEDIZINISCHEN BILDES

DISPOSITIF ET PROCÉDÉ DE RECONSTRUCTION D'IMAGE MÉDICALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2015 KR 20150037639**

(43) Date of publication of application:
**24.01.2018 Bulletin 2018/04**

(73) Proprietors:
• **Vatech Co., Ltd.**
**Hwaseong-si, Gyeonggi-do 445-170 (KR)**
• **Vatech Ewoo Holdings Co., Ltd.**
**Hwaseong-si, Gyeonggi-do 445-170 (KR)**

(72) Inventors:
• **IM, Se Yeol**
**Hwaseong-si**
**Gyeonggi-do 445-170 (KR)**
• **KIM, Tae Woo**
**Hwaseong-si**
**Gyeonggi-do 445-170 (KR)**
• **SEO, Dong Wan**
**Hwaseong-si**
**Gyeonggi-do 445-170 (KR)**
• **CHOI, Sung Il**
**Hwaseong-si**
**Gyeonggi-do 445-170 (KR)**
• **HAN, Tae Hee**
**Hwaseong-si**
**Gyeonggi-do 445-170 (KR)**

(74) Representative: **Markfort, Iris-Anne Lucie**
**Lorenz & Kollegen**
**Patentanwälte Partnerschaftsgesellschaft mbB**
**Alte Ulmer Straße 2**
**89522 Heidenheim (DE)**

(56) References cited:
JP-A- 2007 130 240      KR-A- 20100 070 822
US-A1- 2008 232 539     US-A1- 2009 052 617
US-A1- 2011 026 671     US-A1- 2013 022 252
US-A1- 2015 036 790

• FARMAN ET AL: "Development of imaging
selection criteria and procedures should precede
cephalometric assessment with cone-beam
computed tomography", AMERICAN JOURNAL
OF ORTHODONTICS AND DENTOFACIAL
ORTHOPE, MOSBY, ST. LOUIS, MO, US, vol. 130,
no. 2, 1 August 2006 (2006-08-01), pages 257-265,
XP005592973, ISSN: 0889-5406, DOI:
10.1016/J.AJODO.2005.10.021

**Description**

**Technical Field**

The present invention relates to a device of reconstructing a medical image. More particularly, the present invention relates to a device of reconstructing a panoramic image of a dental arch using image data of a computed tomograhy (CT) image.

**Background Art**

In general, 3D medical images such as a CT image, an ultrasonic image, etc. are capable of generally and clearly examining an examinee. However, in order to observe an internal structure of the examinee in more detail, it may be more effective to use a two-dimensional medical image rather than a three-dimensional medical image. Accordingly, when a 3D medical image and a 2D medical image are displayed at the same time, a user may generally and clearly observe the examinee at the same time.

A conventional example of displaying a 3D medical image and a 2D medical image is disclosed in Korean Patent Application No. 10-2013-0138612 (Publication Date: 19 December 2013), and is as below.

A conventional device for displaying a 3D medical image and a 2D medical image includes: a 3D image obtaining unit obtaining a 3D medical image of an examinee; a cross-section selecting unit selecting at least one cross-section of the examinee based on an external input for the obtained 3D medical image; a 2D image obtaining unit obtaining a 2D medical image by scanning the examinee corresponding to the selected at least one cross section; and a displaying unit displaying unit displaying the 3D medical image and the 2D medical image.

Herein, the cross-section selection unit includes: a window generator generating at least one window on which the obtained 3D medical image is disposed; a window controller moving the at least one generated window in the 3D medical image; and an additional cross-section selector additionally selecting at least one cross-section adjacent to the at least one selected cross-section.

In addition, the 2D image obtaining unit includes: a first image obtainer obtaining at least one first 2D medical image by scanning the examinee according to the at least one selected cross-section; a second image obtainer obtaining at least one second 2D medical image by scanning the examinee according to a cross-section adjacent to the at least one selected cross-section; and a combined image obtainer obtaining at least one combine 2D medical image by combining the at least one first 2D medical image and the at least one second 2D medical image.

In the conventional technique, at least one combined 2D medical image may be obtained by respectively obtaining first and second 2D medical images according to at least one cross-section and at least one cross-section adjacent to the cross-section, and by combining the first and second 2D medical images. However, the combined 2D medical image obtained in this manner differs from an actually radiographed 2D medical image, particularly, from a panorama image.

This is because, a general panorama image is an image in which an image for a desired focus layer within a dental arch trajectory is displayed by shifting and adding an X-ray beam by a uniform interval according to the dental arch trajectory. However, when a 2D medical image is obtained by adding medical images within a selected area (range) using a simple image combining method such as method used in conventional technique, it causes a problem that an inaccurate 2D medical image is obtained.

Meanwhile, when radiographing, in many cases, radiographing may be performed while a patient (examinee) is not posing accurately for the radiographing according to a skill of a radiographer.

Thus, a CT image is radiographed in a state in which the patient is posing in a wrong posture may not prevent a situation in which bilateral symmetry and facial skeleton structures appear to be misaligned.

Accordingly, when a CT image obtained in a state in which a posture of the patient is not accurate when radiographing is displayed through a medical diagnosing 3D viewer, there is a problem the CT image looks distorted.

Meanwhile, conventionally, a panorama image is generated by reconstructing in a manual method from a CT image. In other words, conventionally, a user manually and arbitrarily inputs a dental arch trajectory in an image of an axial cross-section among CT images, and a panorama image is generated by reconstructing image data of an arbitrary area that is orthogonal to the dental arch trajectory that is manually input. Herein, reconstructed the panorama image is generated after applying the dental arch trajectory that is designated by manually inputting by the user in a specific cross-section to all cross-sections.

However, since each cross-section has a different position, when a tooth is present outside the dental arch trajectory when reconstructing a panorama image using a manual method, the corresponding tooth does not appear in the panorama image.

From Farman et al: "Develoment of imaging selction criteria and procedures should precede cephalometric assessment with cone-beam computed tomography" (American Journal of Orthodontics and dentofacial orthope, Mosby, St. Louis, MO, US, col. 130. No. 2, 01. 08.2006, pages 257-265, XP005592973, ISSN: 0889-5406, DOI: 10.1016/J.AJODO. 2005.10.021) a solution is known according to the preamble of claim 1. Moreover, reference is made as well to US 2011/026671 A1 and US 2009/052617 A1 disclosing further known solutions in the technical field.

**Disclosure**

**Technical Problem**

Accordingly, the present invention has been made to solve the above-mentioned problems occurring in the prior art.

Accordingly, an object of the present invention is to provide a device of reconstructing a medical image for generating a 2D medical image using an automatic reconstruction method by correcting a misalignment generated by an accurate posture of a patient when radiographing by using a pre-radiographed 3D medical image so that a corresponding image is correctly positioned at a 3D space without additionally radiographing a 2D medical image (for example: a panorama image), and by detecting a dental arch trajectory and generating the 2D medical image based on the detected dental arch trajectory.

The objectives of the present invention are not limited to those mentioned above. Other objectives and advantages of the present invention which are not disclosed will be understood from the following description, and be apparent with reference to the embodiments of the present invention. Also, it is obvious to those skilled in the art that the objectives and advantages of the present invention will be realized by the means as claimed and combinations thereof.

**Technical Solution**

A device of the present invention for accomplishing the above object includes the features of claim 1. Further features of the invention are described in claims 2 to 5 and the following description.

**Advantageous Effects**

According to the present invention as described above, a panorama image is generated by correcting a misalignment generated by an accurate posture of a patient when radiographing by using a pre-radiographed 3D medical image so that a corresponding image is correctly positioned in a 3D space, and by detecting a dental arch trajectory and generating the 2D medical image using an automatic reconstruction method based on the detected dental arch trajectory, thus there is no need for additionally radiographing a panorama image, and an exposure dose of an examinee (patient) may be reduced by removing additional exposure.

In addition, in the present invention, user convenience and image quality may be improved compared with a conventional method that reconstructs a panorama image using a manual method.

In addition, in the present invention, a positional accuracy for each cross-sectional image may be improved by correcting a misalignment generated by an accurate posture of a patient when radiographing. Accordingly, it is possible to make an accurate medical diagnosis.

In addition, in the present invention, a misalignment of a facial skeleton structure in an image may be prevented by applying a CT geometry correction by using CT data when automatically generating a panorama image using an automatic reconstruction method. Accordingly, a positional accuracy for each cross-sectional image and an enlarged image may be improved.

In addition, the present invention does not require a separate or additional device configuration for implementing a panorama image radiographing device, thus a size of the medical device and an installation space thereof may be reduced.

**Description of Drawings**

FIG. 1 is a configuration diagram of a medical image reconstruction device according to an embodiment of the present invention.
FIG. 2 is a detailed configuration diagram of a geometry correction unit according to the embodiment of the present invention.
FIG. 3 is a detailed configuration diagram of a correction angle extractor according to the embodiment of the present invention.
FIG. 4 is a detailed configuration diagram of a rotation angle count map extractor according to the embodiment of the present invention.

FIG. 5 is a detailed configuration diagram of a dental arch trajectory generator according to the embodiment of the present invention.

FIG. 6 is a view showing an extraction of an overlapped image having a value satisfying threshold values t1 and t2.

FIG. 7 is a view showing a positional coordinate detection method of X and Y axis of an anterior teeth occlusion surface.

FIG. 8 is a view showing an average image and a gradient image of the anterior teeth occlusion surface.

FIG. 9 is a view showing an angle extraction image in the gradient image.

FIG. 10 is a view showing a detection area for calculating a fixed dental arch coordinate of a 45° angle.

FIG. 11 is a view showing a dental arch coordinate extraction method using a histogram distribution of the detection area.

FIG. 12 is a view showing a detection area for calculating a fixed dental arch coordinate of a 90° angle.

FIG. 13 is a view showing a dental arch coordinate extraction method using a histogram distribution of the detection area.

FIG. 14 is a view showing a detection area for calculating an anterior dental arch coordinate.

FIG. 15 is a view showing an anterior dental arch coordinate extraction method using a histogram of the detection area.

FIG. 16 is a flowchart of a medical image reconstruction method according to an embodiment of the present invention.

FIG. 17 is a detailed flowchart of a geometry correction according to the embodiment of the present invention.

FIG. 18 is a detailed flowchart of a correction angle extraction according to the embodiment of the present invention.

FIG. 19 is a detailed flowchart of a rotation angle count map extraction according to the embodiment of the present invention.

FIG. 20 is a detailed flowchart of a dental arch trajectory generation 620 according to the embodiment of the present invention.

## Mode for Invention

The above and other objects, features, and advantages of the present invention can be appreciated by the following description and will be understood more clearly by an embodiment of the present invention. In addition, it will be appreciated that the objects and advantages of the present invention will be easily realized by means shown in the appended patent claims. Accordingly, the present invention can be easily implemented by one of ordinary skill in the art. Further, if it is determined that the detailed description of the known art related to the present invention makes the gist of the present invention unnecessarily obscure, a detailed description thereof will be omitted.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Also, when a part may "include or comprise" a certain constituent element, unless specified otherwise, it may not be construed to exclude another constituent element but may be construed to further include other constituent elements.

Hereinafter, preferred embodiments of the present invention will be described in detail with reference to the accompanying drawings.

First, technical points according to an embodiment of the present invention will be summarized as follows.

In the embodiment of the present invention, a panorama image is generated using an automatic reconstruction method by applying a CT geometry correction to CT data so that a CT image is correctly positioned at a three-dimensional space, and by using the corrected CT image.

Herein, in the embodiment of the present invention, a positional coordinate for each position of an X-ray source of a dental arch trajectory is set by using a dental arch trajectory detected by using teeth feature information in a CT image, and a rotate center point (RCP) coordinate calculated by using radiograph sequence information of a device. A panorama image is generated by reconstructing image data of an intersection part between an X-ray irradiation path based on each positional coordinate and dental arch trajectory.

In other words, in the embodiment of the present invention, first, in order to correct a misalignment generated by an inaccurate posture of a patient while radiographing, a positional misalignment is corrected by applying a CT geometry correction algorithm. Herein, an optimized position is calculated by using a feature value of a region of interest (ROI) designated in the CT image. Then, a dental arch trajectory is detected by applying a dental arch trajectory detection algorithm using teeth feature values of the CT image. An RCP coordinate is calculated by using the dental arch trajectory and the device radiograph sequence information. Next, an automatic reconstruction algorithm is applied thereto to generate a panorama image in the CT image. Herein, a positional coordinate for each position of an X-ray source of a dental arch trajectory is set by using the detected dental arch trajectory and the RCP coordinate, and the panorama image is generated by performing a summation of the X-ray irradiation path generated from each positional coordinate and image data of the intersection areas between dental arch trajectory and the X-ray irradiation path, and arranging the summation result according the dental arch trajectory.

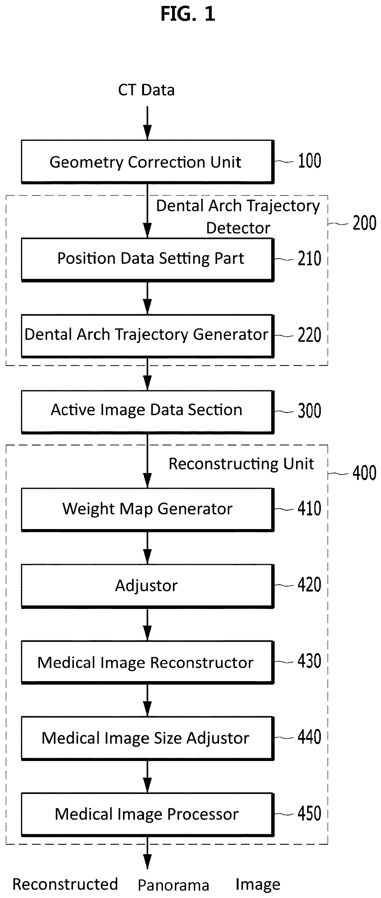

FIG. 1 is a configuration diagram of a medical image reconstruction device according to an embodiment of the present invention.

As shown in FIG. 1, the medical image reconstruction device according to the embodiment of the present invention includes: a geometry correction unit 100 correcting so that a CT image is correctly positioned at a three-dimensional space by applying a CT geometry correction to CT data; a dental arch trajectory detection unit 200 detecting a dental arch trajectory; an active section setting unit 300 setting an active image data section and an X-ray irradiation path; and a reconstructing unit 400 implementing a panorama image by performing a summation of image data of the CT image corrected in the geometry correction unit 100 within the "active image data section set in the active section setting unit 300" based on the dental arch trajectory detected in the dental arch trajectory detection unit 200 for each X-ray irradiation path, and reconstructing the summation result according the dental arch trajectory.

Next, detailed descriptions of respective components will be described as follows.

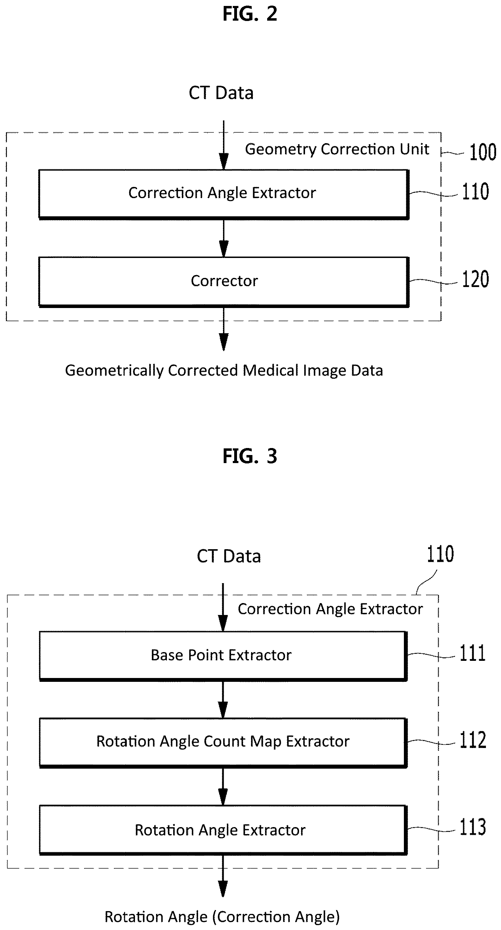

First, the geometry correction unit 100 performs a CT geometry correction to correct a misalignment generated by an inaccurate posture of a patient when radiographing. Herein, an image after applying the CT geometry correction has a form in which a face of the patient is facing the front, a bilaterally symmetric organ such as chin, eyes, etc., or feature points maintain a horizontal level, and is a transformed image so that the head is not bent too much or is held. The geometry correction unit 100 will be described in more detail with reference to FIGS. 2 to 4.

FIG. 2 is a detailed configuration diagram of the geometry correction unit 100 according to the embodiment of the present invention.

As shown in FIG. 2, the geometry correction unit 100 according to the embodiment of the present invention includes: a correction angle extractor 110 receiving CT data and extracting a correction angle for each cross-sectional direction for a positional correction of the CT data; a corrector 120 performing a geometry correction by rotating a CT image by using the correction angle for each cross-sectional direction extracted in the correction angle extractor 110.

Next, detailed descriptions of respective components will be described as follows.

First, a CT radiograph device (not shown in the figure) radiographs, for example, a head part of an examinee and transmits CT data to a geometry correction device of medical image data.

Then, the correction angle extractor 110 receives the CT data from the CT radiograph device, and extracts a correction angle for each cross-sectional direction (coronal, sagittal, axial) of a multi planar reconstruction (MPR) for the positional correction of the CT data. Herein, in order to extract the correction angle for each cross-sectional direction of the MRI, a sub-routine (correction angle extractor of FIG. 3) is performed for each cross-sectional direction. Herein, the MRI means reconstructing a number of 2D cross-sectional images from a 3D image. Coronal, sagittal, and axial cross-sections are related with each cross section of X, Y, and Z axis of the 3D image.

In addition, the corrector 120 performs a geometry correction by rotating the CT image by using a rotation angle (correction angle) for each cross-sectional direction extracted in the correction angle extractor 110. Herein, the rotation angle (correction angle) may be calculated as three parameters according to coronal, sagittal, and axial directions.

FIG. 3 is a detailed configuration diagram of the correction angle extractor 110 according to the embodiment of the present invention.

As shown in FIG. 3, the correction angle extractor 110 according to the embodiment of the present invention includes: a base point extractor 111 extracting a base point in the CT data; a rotation angle count map extractor 112 extracting a count map for each preset rotation angle by using image data of an ROI including the base point extracted from the base point extractor 111; and a rotation angle extractor 113 extracting a rotation angle of a minimum count map among the count maps for respective rotation angles extracted in the rotation angle count map extractor 112.

Next, detailed descriptions of respective components will be described as follows.

First, the base point extractor 111 receives the CT data from the CT radiograph device, and extracts any one piece of the base point which may be extracted within the CT data. Herein, as the base point data, jawline data, teeth arrangement data, base point data with an ear plug, positional data of a temporomandibular joint (TMJ), eye position data, etc. may be included.

For example, when jawline data of a lower jaw (coordinate at three-dimensional space) is used as the base point, an overlapped image is obtained by overlapping an axial cross-sectional image of a designated area within the CT image. When obtaining the overlapped image, an arbitrary threshold value that will be described later is used for extracting the jawline data of the lower jaw. Herein, the Formula for generating the overlapped image is as the [Formula 1] below.

[Formula 1]

$$g(x,y) = \sum_{z=zs}^{ze} \sum_{y=0}^{h-1} \sum_{x=0}^{w-1} f(x,y,z)$$

Herein, x, y, and z mean positional values of X, Y, and Z axis, w is a horizontal size of a cross-sectional image, and h is a vertical size of a cross-sectional image. zs is a starting point that is set in an Z axis for overlapping, and ze is an ending point that is set in the Z axis for overlapping. Starting point and ending point values for overlapping a CT image may vary since the values may be changed according to a characteristic of a patient, and may be used by optimizing through an experiment. The corresponding example is as the [Formula 2] below.

[Formula 2]

$$zs = z - 20$$
$$ze = zs - (z * 0.45)$$

In addition, the Formula of an overlapped image generation condition is as the [Formula 3] below.

[Formula 3]

$$f(x,y,z) = \{g(x,y) + 1, \qquad t1 < I(x,y,z) < t2\}$$

Herein, I(x,y,z) means each pixel value of the CT image, when a designated threshold value is satisfied, a value of an overlapped image g(x,y) is increased. A designated threshold value t1 is 1000, and t2 is 2000. Herein, the threshold values t1 and t2 may vary since the values may be changed according to the CT radiograph device or a radiograph condition, and may be used by optimizing through an experiment.

In addition, a central area of the jawline of the lower jaw is calculated by using overlapped image data. Image data of the central area of the jawline of the lower jaw is extracted within an axial cross-sectional image of a designated section in the CT image. When the extracted image data satisfies a condition that will be described later, a coordinate of a cross-section of the extracted image data is used as the central area of the lower jaw jawline. The Formula h(x,y) for detecting the central area of the jawline of the lower jaw is as the *[Formula 4] below.

[Formula 4]

$$h(x,y) = \sum_{z=zs}^{ze} \sum_{y=by-sy}^{by+sy} \sum_{x=bx-sx}^{bx+sx} j(x,y,z)$$

Herein, bx and by are a central coordinate of a section designated for detecting the central area of the jawline of the lower jaw within the overlapped image, sx and sy are size values of the corresponding section. Herein, the Formula for setting the corresponding section is as the [Formula 5] below.

[Formula 5]

$$bx = \frac{1}{2}\left( \sum_{y=0}^{h-1} \sum_{x=0}^{w-1} X(x,y) + \sum_{y=0}^{h-1} \sum_{x=w-1}^{0} X(x,y) \right)$$

$$by = \sum_{y=0}^{h-1} \sum_{x=0}^{w-1} Y(x,y)$$

In addition, the Formulas X(x,y) and Y(x,y) for detecting an X-axis central coordinate of the designated section is as [Formula 6] below.

$$[\text{Formula 6}]$$

$$X(x,y) = \{x, \qquad g(x,y) > t\}$$
$$Y(x,y) = \{y, \qquad bx \neq 0\}$$

In order to set a valid point within the overlapped image, an arbitrary threshold value t is used as a base. For example, the threshold value t is 40. Herein, a value of the threshold value t may vary since the value may be changed according to an image characteristic, and may be used by optimizing through an experiment.

For example, all of area size values sx and sy for setting of the section based on the detected central coordinate are 100. Herein, the area size values may be varied according to an image characteristic, and may be used by optimizing through an experiment.

Finally, the Formula j(x,y,z) for detecting jawline information of the lower jaw within the designated section is as [Formula 7] below.

$$[\text{Formula 7}]$$

$$j(x,y,z) = \{z, \qquad TC = m * m\}$$

Herein, m means a mask size, a total number TC in which a pixel value satisfies the threshold values t1 and t2 by retrieving a mask area adjacent to each pixel within the designated area is calculated as [Formula 8] below.

$$[\text{Formula 8}]$$

$$TC = \sum_{my=y-m/2}^{y+m/2} \sum_{mx=x-m/2}^{x+m/2} k(mx, my)$$

$$k(mx, my) = \{TC + 1, \qquad t1 < I(x,y,z) < t2\}$$

$$t1 = 1000$$
$$t2 = 2000$$

Herein, when a TC value is equal to a size of the mask area, a corresponding positional point is used as a positional coordinate of the jawline of the lower jaw.

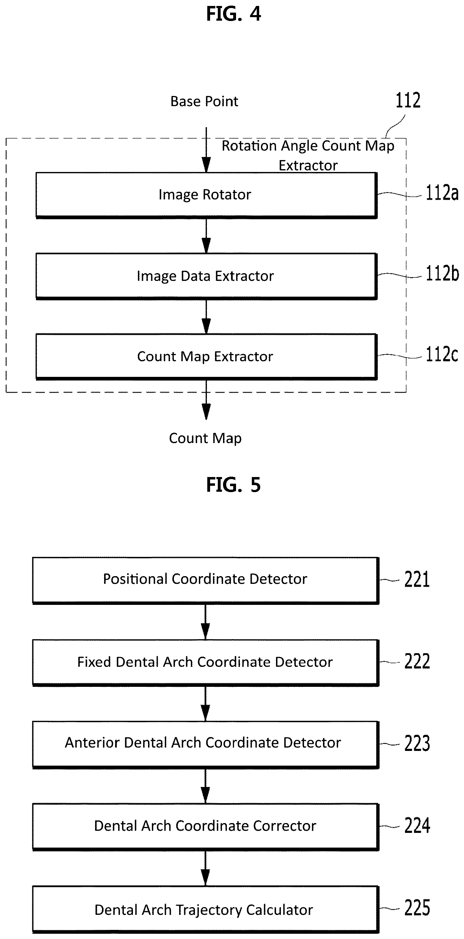

In addition, the rotation angle count map extractor 112 extracts a count map for each preset rotation angle by using image data of an ROI extracted based on the base point extracted in the base point extractor 111. Herein, a subroutine (rotation angle count map extractor of FIG. 4) is performed for each rotation angle to extract the count map for each rotation angle.

In addition, the rotation angle extractor 113 extracts a rotation angle of the count map having the least pixels (in other words, the minimum count map) by measuring a number of valid pixels of the count map for each rotation angle extracted in the rotation angle count map extractor 112.

FIG. 4 is a detailed configuration diagram of the rotation angle count map extractor 112 according to the embodiment of the present invention.

As shown in FIG. 4, the rotation angle count map extractor 112 according to the embodiment of the present invention includes: an image rotator 112a rotating the CT image for each preset rotation angle; an image data extractor 112b extracting image data of an ROI based on the base point extracted in the base point extractor 111 from the CT image rotated in the image rotator 112a; and a count map extractor 112c extracting a count map by using the image data of the ROI extract in the image data extractor 112b.

Next, detailed descriptions of respective components will be described as follows.

First, the image rotator 112a repeatedly rotates the CT image by a pre-designated (set) rotation angle range with a preset arbitrary rotation angle interval (for example: 0.1 degree). Herein, the rotation angle interval may be arbitrarily changed, may be set with an interval in addition to the 0.1 degree interval, or may be used by setting according to a trade-off relation between a processing time and accuracy.

In addition, in order to improve a processing speed, an image rotation process may be performed by scaling down the CT image. For example, original information of 800*800*600 size may be processed by scaling down as 400*400*600, 400*400*300, 400*800*300, etc. In addition, in order to accurately calculate the rotation angle for an image correction, the image rotation process may be performed after applying a pre-process algorithm such as a noise removing filter, etc.

For example, the rotation angle range of the designated CT image for each cross-section may be set as (-10°~ 10°) for axial, (-9°~ 9°) for sagittal, and (-3°~ 3°) for coronal, and the above example corresponds to one example.

In addition, setting of the rotation angle may be used by parameterizing according to each device and considering a range in which CT radiograph output information may be damaged.

In addition, the image rotation may be performed by repeating operations of the image data extractor 112b and the count map extractor 112c which will be described later while an angle is changed by the preset rotation angle interval (for example: 0.1 degree) according to the axial direction, the sagittal direction, and coronal direction.

In addition, an order of image rotation may be arbitrarily selected, a method of finding a rotation angle by determining count maps for all directions may be possibly used, or a method of finding a rotation angle by sequentially determining count maps of the axial direction, the sagittal direction, and the coronal direction may be used.

In addition, the image data extractor 112b extracts image data of the ROI designated for each cross-section based on the jawline data of the lower jaw (base point) extracted in the base point extractor 111 from the CT image rotated in the image rotator 112a. Herein, the ROI may be set to a size including both of the upper jaw and the lower jaw. Herein, setting of the ROI may be variably set in addition to a form including both of the upper jaw and the lower jaw since the ROI is a factor that may affect the performance of the device (or algorithm) according to the embodiment of the present invention.

In addition, the count map extractor 112c extracts a count map by using the image data within the ROI extracted in the image data extractor 112b based on a designated threshold value. Herein, the Formula g(x,y) for generating the count map is as the [Formula 9] below.

$$[Formula\ 9]$$

$$g(x,y) = \sum_{z=r1}^{r2} \sum_{y=0}^{h-1} \sum_{x=0}^{w-1} f(x,y,z)$$

Herein, r1 and r2 are height values of the designated ROI.

In addition, the Formula f(x,y,z) for a count map generation condition is as the [Formula 10] below.

$$[Formula\ 10]$$

$$f(x,y,z) = \{1, \qquad t1 < I(x,y,z) < t2\}$$

Herein, the designated threshold value t1 may be 500 and t2 may be 1500, or may be set by varying according to the CT radiograph device or a radiograph environment.

Meanwhile, the dental arch trajectory detection unit 200 includes: a position data setting part 210 setting a standard dental arch and a rotate center point (RCP) coordinate by using the device radiograph sequence information; and a dental arch trajectory generator 220 generating a dental arch trajectory by using the standard dental arch set in the position data setting part 210.

Herein, the position data setting part 210 sets the standard dental arch and the RCP coordinate by using the device radiograph sequence information based on a positional coordinate of an anterior teeth occlusion surface.

The set standard dental arch and the RCP coordinate are used when calculating an X-ray path for each position of the dental arch trajectory. A panorama image is implemented by performing a summation of image data of a designated section based on a point in which the X-ray path and a dental arch trajectory intersect, and by reconstructing the summation result according to the dental arch trajectory. Herein, the X-ray path for the summation is determined according to positions of the standard dental arch and the RCP coordinate. The X-ray path may be determined by calculating where X-ray is generated at an X-ray source position and each voxel positions of a CT radiograph which the X-ray passes through considering radiographing with an actual panorama image radiograph device.

For example, considering a voxel size of the CT data, the positions of the standard dental arch and the RCP coordinate are set after moving by 8mm in a Y axis based on the positional coordinate of the anterior teeth occlusion surface. An optimized value that is calculated through an experiment may be applied to such a positional moving distance of the standard dental arch and the RCP coordinate. The 8mm value is one example that is set by considering a position to minimize scaling up/down of the reconstructed panorama image. Accordingly, such a value may vary, and may be applied by parameterizing.

In addition, the dental arch trajectory generator 220 uses a cubic-spline curve to generate the dental arch trajectory by using the standard dental arch set in the position data setting part 210. The dental arch trajectory generator 220 according to the embodiment of the present invention will be described in more detail with reference to FIGS. 5 to 15.

FIG. 5 is a detailed configuration diagram of the dental arch trajectory generator 220 according to the embodiment of the present invention.

As shown in FIG. 5, the dental arch trajectory generator 220 according to the embodiment of the present invention includes: a positional coordinate detector 221 detecting a positional coordinate of the anterior teeth occlusion surface; a fixed dental arch coordinate detector 222 detecting a fixed dental arch coordinate based on the positional coordinate of the anterior teeth occlusion surface detected in the positional coordinate detector 221; an anterior dental arch coordinate detector 223 detecting an anterior dental arch coordinate for each cross-section based on the positional coordinate of the anterior teeth occlusion surface detected in the positional coordinate detector 221; a dental arch coordinate corrector 224 correcting a dental arch coordinate by using the fixed dental arch coordinate detected in the fixed dental arch coordinate detector 222 and the anterior dental arch coordinate detected in the anterior dental arch coordinate detector 223; and an dental arch trajectory calculator 225 calculating a dental arch trajectory by using the dental arch coordinate that is corrected and detected for each cross-section in the dental arch coordinate corrector 224.

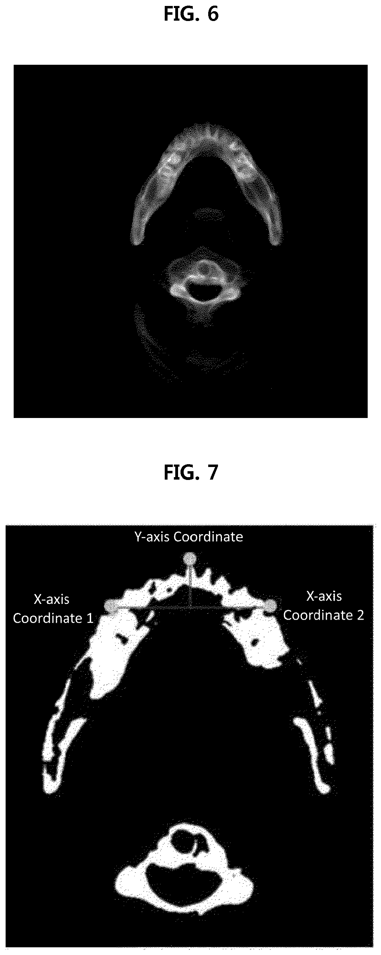

Herein, the positional coordinate detector 221 extracts an overlapped image having a value satisfying specific threshold values t1 and t2 to a cross-section of a designated height based on an end position of the lower jaw (Refer to FIG. 6). For example, the threshold value t1 may be 2000 and the t2 may be 2000, and may be set by varying according to the CT radiograph device or a radiograph environment. Herein, when the positional coordinate of the anterior teeth occlusion surface is detected in the extracted overlapped image, a noise filter may be applied to improve the accuracy.

Describing with reference to FIG. 7, in order to detect a point where a high degree of overlaying of a tooth in the extracted overlapped image, a Y-axis coordinate satisfying a specific threshold value t3 is extracted. For example, the threshold value t3 may be 50, and may be set by varying according to the CT radiograph device or a radiograph environment.

Extracting the starting and ending points of X-axis at a distance of 1 to 1.5cm relative to the Y axis, and calculate the intermediate value to extract the X axis coordinates. An experimentally optimized value may be applied to a Y-axis base moving distance. The 1~1.5cm value is an example which is set by considering a form of a dental arch, and may be applied by parameterizing.

An Z-axial coordinate is arbitrarily set at 3.5 cm upper point based on the jaw end positional coordinate. Herein, 3.5cm is comes from an experimental result value, statistically, the distance from the end of the jaw to the occlusion surface is 2.4 cm for children and 4.0 cm for adults. Accordingly, the optimized distance set value may be varied by considering a patient's physique or age, and may be applied by parameterizing.

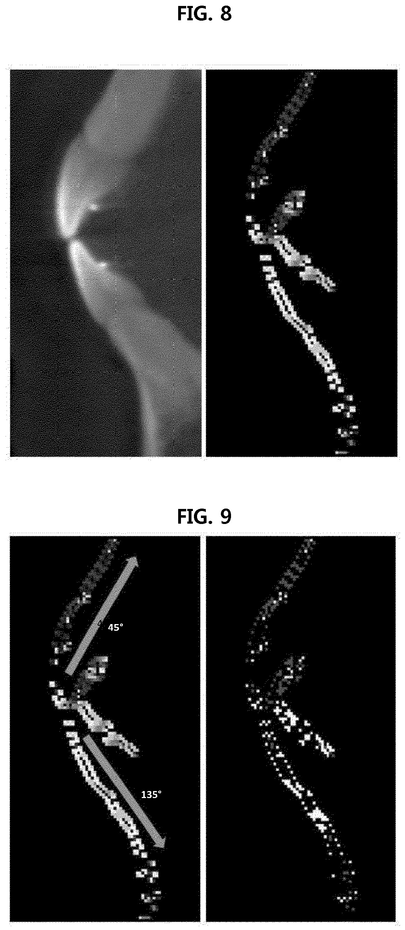

Describing with reference to FIG. 8, an image is generated with an average value of each pixel within the ROI by using the positional coordinate of the anterior teeth occlusion surface which is extract first, and a gradient image is extracted from the average image. Herein, the ROI means an area having a square form including the anterior teeth, and a value for setting a size of the ROI may be used by parameterizing.

As shown in FIG. 9, a tooth angle between the upper jaw and the lower jaw within the extracted gradient image is calculated. The Formula A(x,y) for calculating the tooth angle is as the [Formula 11] below.

$$[Formula\ 11]$$

$$A(x,y) = \sum_{y=0}^{ry} \sum_{x=0}^{ry} \tan^{-1}(G(x,y) \times (180 \div \pi))$$

Herein, G(x,y) represents a pixel value of the gradient image.

In order to improve calculation efficiency, arbitrarily, angles, except for 45° and 135°, are set to 0. This is because the upper jaw is highly distributed at a 45° angle, and the lower jaw at a 135° angle.

Herein, in order to detect the position of the tooth occlusion surface, a number of pieces of angle information distributed in a specific area of the image in which the tooth angle is calculated is detected, a point having the least difference in angular number of 45° and 135° is extracted. The corresponding extracted point is used as the positional coordinate of the anterior teeth occlusion surface.

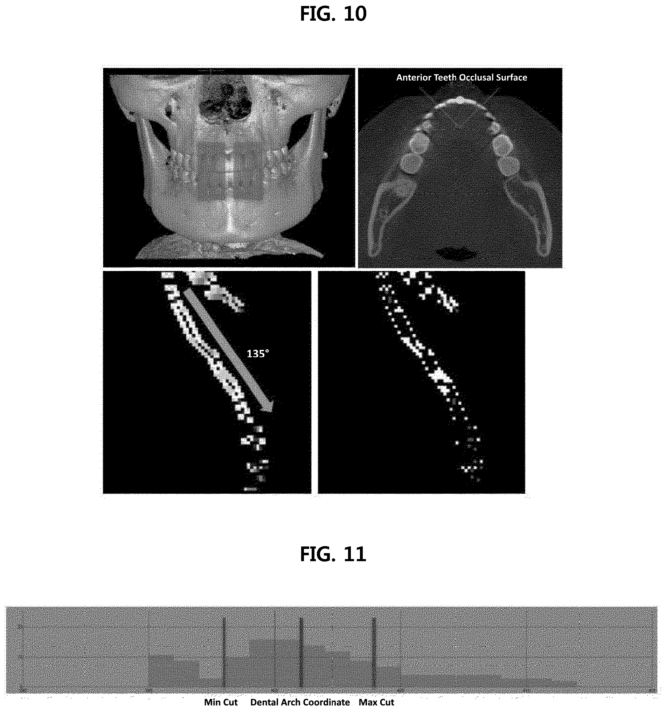

In addition, the fixed dental arch coordinate detector 222 generates a histogram satisfying threshold values t1 and t2 of a detection area (refer to FIG. 10) that is designated as right/left 45° after moving by 1.5cm in the Y axis based on the positional coordinate of the anterior teeth occlusion surface detected in the positional coordinate detector 221. A value that is calculated by optimizing through an experiment may be applied to the Y axial based moving distance. The 1.5cm value is one example which is set by considering a form of a dental arch, and may be changed and applied by parameterizing. For example, the threshold value t1 may be 1000 and the t2 may be 2000, and may be set by varying according to the CT radiograph device or a radiograph environment.

As shown in FIG. 11, a central point after cutting the minimum and maximum 20% from the total number of histograms is extracted as the dental arch coordinate.

As described above, the fixed dental arch coordinate of 45° is extracted in the positional coordinate of the anterior teeth occlusion surface, and the corresponding coordinate is used in all cross-sections.

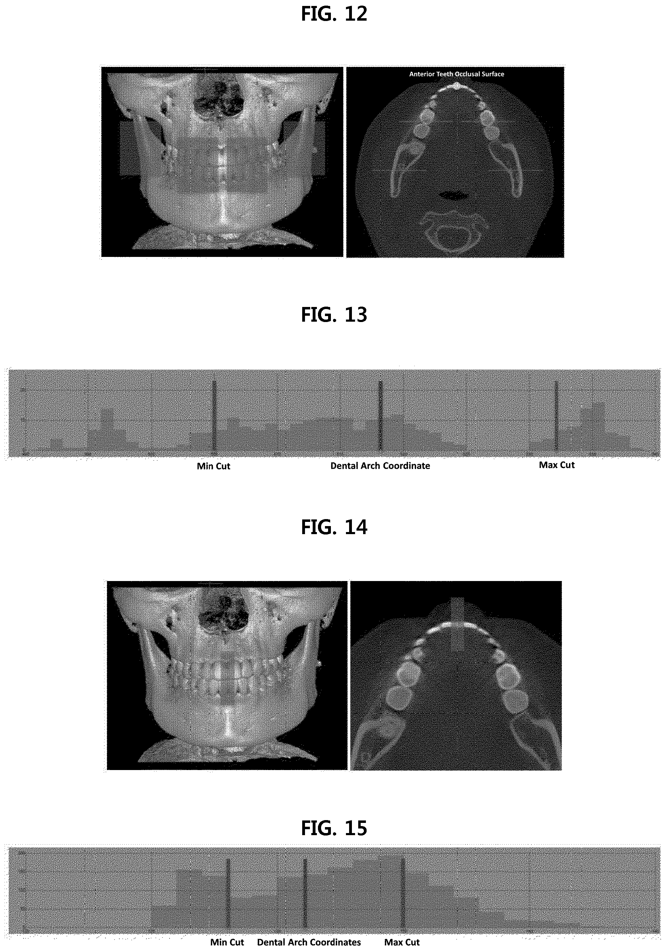

In addition, the fixed dental arch coordinate detector 222 generates a histogram that satisfies threshold values t1 and t2 of a detection area designated as right/left 90° after moving by 2.5cm and 6.5cm in the Y axis based on the positional coordinate of the anterior teeth occlusion surface detected in the positional coordinate detector 221 (refer to FIG. 12). A value that is calculated by optimizing through an experiment may be applied to a Y-axis base moving distance. The 2.5cm and 6.5cm values are one example which are set by considering a form of a dental arch, and may be changed and applied by parameterizing. For example, the threshold value t1 may be 1000 and t2 may be 2000, and may be set by varying according to the CT radiograph device or a radiograph environment.

As shown in FIG. 13, a central point after cutting the minimum and maximum 20% from the total number of histograms is extracted as the dental arch coordinate.

As described above, the fixed dental arch coordinate of 90 degrees is extracted in the positional coordinate of the anterior teeth occlusion surface, and the corresponding coordinate is used in all cross-sections.

In addition, the anterior dental arch coordinate detector 223 generates a histogram that satisfies threshold values t1 and t2 of a detection area designated based on the positional coordinate of the anterior teeth occlusion surface detected in the positional coordinate detector 221 (refer to FIG. 14). For example, the threshold value t1 may be 1000 and t2 may be 2000, and may be set by varying according to the CT radiograph device or a radiograph environment.

As shown in FIG. 15, a central point after cutting the minimum and maximum 20% from the total number of histograms is extracted as the dental arch coordinate.

Extracting of the anterior dental arch coordinate described above is repeatedly performed for each cross-section.

In addition, When the fixed dental arch coordinates detected by the fixed dental arch coordinate detection unit 222 is located at the upper side on the Y-axis than the anterior dental arch coordinate detected by the anterior dental arch detecting unit 223, the dental arch coordinate corrector 224 corrects the position of the fixed dental arch coordinate at 1 mm below the anterior dental arch to prevent a deformation of a curve of the dental arch trajectory. Herein, an experimentally optimized value may be applied as the correction distance. The 1mm value is one example which is set by considering a form of a dental arch, and may be changed and applied by parameterizing.

Correcting of the dental arch coordinate described above is repeatedly performed for each cross-section.

In addition, the dental arch trajectory calculator 225 uses a cubic-spline curve as the [Formula 12] below to calculate the dental arch trajectory by using the dental arch coordinate corrected and detected for each cross-section through the dental arch coordinate corrector 224.

[Formula 12]

$$B(t) = P_0(1-t)^3 + 3P_1 t(1-t)^2 + 3P_2(1-t) + P_3 t^3, \quad t \in [0,1]$$

In addition, the dental arch trajectory calculated by using the cubic-spline curve is divided into a uniform interval corresponding to a voxel size of a CT radiograph volume, or may be divided by using the device radiograph sequence information. Herein, the division is performed by moving to left and right directions based on a central point of the detected dental arch trajectory.

Meanwhile, in order to set the active image data section, the active section setting unit 300 calculates an orthogonal section coordinate which is located in a pre-determined distance from the standard dental arch by calculating an intersection point between the trajectory of the standard dental arch set in the position data setting part 210 and the dental arch trajectory detected in the dental arch trajectory detection unit 200. Herein, the corresponding orthogonal section coordinate orthogonally crosses a slope of the standard dental arch and the RCP coordinate. In addition, since molar teeth and the anterior teeth are different in thickness and reference intervals, a summation section (active image data section) may be differently applied. The summation section of the anterior and the molar teeth may be set by using a pre-determined value by considering the corresponding characteristic.

In addition, the active section setting unit 300 calculates an X-ray irradiation path for each position of the dental arch trajectory, and a positional coordinate of the X-ray based on the RCP coordinate.

Meanwhile, the reconstructing unit 400 includes: a weight map generator 410 generating a weight map; an adjustor 420 adjusting a ratio of image that is reflected on a reconstructing panorama image; and a medical image reconstructor 430 reconstructing a panorama image by performing a summation of "the image data within the active image data section set in the active section setting unit 300" and which is adjusted in the adjustor 420 according to the weight value of the weight map generator 410.

Herein, the reconstructing unit 400 further includes an image size adjustor 440 adjusting a size of the panorama image reconstructed in the medical image reconstructor 430.

In addition, the reconstructing unit 400 further includes a medical image processor 450 performing image processing to the panorama image reconstructed in the medical image reconstructor 430.

Herein, in order to improve a tooth feature and visibility, the weight map generator 410 generates a 3D gradient map of the CT radiograph volume by using the dental arch trajectory detected in the CT radiograph volume, and uses the generated 3D gradient map as a weight value when performing the summation.

Herein, the 3D gradient map is extracted for each cross-section (axial, sagittal, coronal) by using a sobel mask as the [Formula 13] below, and considering a characteristic of the X-ray path, gradient information of sagittal and coronal cross-sections is used by combining with each other and by using each path angular information.

$$[Formula\ 13]$$

| Sobel Mask | | Row | | | Column | | |
|---|---|---|---|---|---|---|---|
| | −1 | −2 | −1 | | −1 | 0 | 1 |
| | 0 | 0 | 0 | | −2 | 0 | 2 |
| | 1 | 2 | 1 | | −1 | 0 | 1 |

$$G_{val} = G_{coronal} * (1.0 - W_{angle}) + G_{Sagittal} * W_{angle}$$

In addition, the adjustor 420 generates and uses an opacity table. In other words, considering a characteristic of a CT number, the opacity table is generated by applying a gamma curve to an area with a specific threshold value or less. Herein, the opacity table is a kind of a look-up table, and is used as one method of adjusting a ratio of image that is reflected on the reconstructing panorama image in the present invention. The Formula T(x) for generating the opacity table is as the [Formula 14] below.

$$[Formula\ 14]$$

$$T(x) = \sum_{x=0}^{s} f(x)$$

$$f(x) = \begin{cases} (x/(t-1))^g \times x, & x < t \\ x, & x \geq t \end{cases}$$

Herein, s means a size of the opacity table, and g means a gamma curve power. A threshold value t may be applied by being optimally calculated through an experiment. For example, the threshold value t is set as 2000 considering a CT number.

In addition, the medical image reconstructor 430, among the image data of the CT image corrected in the geometry correction unit 100, performs the summation of the "image data within the active image data section set in the active section setting unit 300" and which is adjusted in the adjustor 420 for each X-ray irradiation path and for each position of the dental arch trajectory according to the weight value of the weight map generator, and reconstructs the panorama image by correctly overlapping or arranging or both the summation result according the dental arch trajectory. Herein, a point in which the orthogonal section of the standard dental arch and the detected dental arch coordinate intersect is calculated, and a summation of image data of a designated section is performed based on the corresponding point.

In addition, when performing the summation, a weight value is applied after applying an active image data section value to the opacity table. Herein, the weight value may be applied by combining a Gaussian weight and a gradient weight, or may be independently applied. In addition, an intensity value of a pixel within the summation section may be used as the weight value.

Herein, when the gradient weight is used, information of the corresponding section is obtained in the 3D gradient map by using a positional coordinate of the summation section. The corresponding section information uses an interpolation result value by applying a bi-Linear interpolation method. Herein, a gradient information value has a characteristic to have a large value nearby an edge, thus smoothing is performed by applying a Gaussian mask to utilize internal information and neighboring information of the edge. In addition, result information is normalized and transformed to a gradient weight. In addition, in order to generate a final weight, the normalized information is combined with the Gaussian weight. Then, the summation is performed by applying the finally generated weight to a result value that has passed the opacity table.

The panorama image is reconstructed by applying the above process to all X-ray path ranges of the dental arch trajectory detected for each cross-section.

In addition, the medical image size adjustor 440 adjusts the size of the reconstructed panorama image in the CT data. In other words, the image size is adjusted by cutting a specific area to derive a result image similar to an image radiographed in the panorama image radiograph device or to remove unnecessary border parts. Herein, when adjusting of the image size is not necessary, the operation of the medical image size adjustor 440 may be omitted.

In addition, the medical image processor 450 improves image quality by performing a post processing to the reconstructed panorama image. In order to maximize the image quality improvement, a pre-processing process may be applied before the geometry correction unit 100.

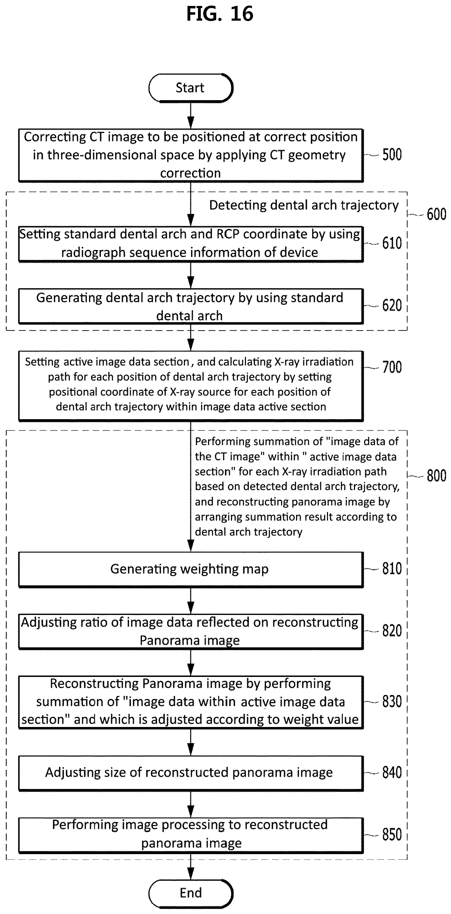

FIG. 16 is a flowchart of a medical image reconstruction method according to an embodiment of the present invention. The detailed embodiment thereof has been described in detail in the description of the medical image reconstruction device. Hereinafter, an operation process thereof will be briefly described.

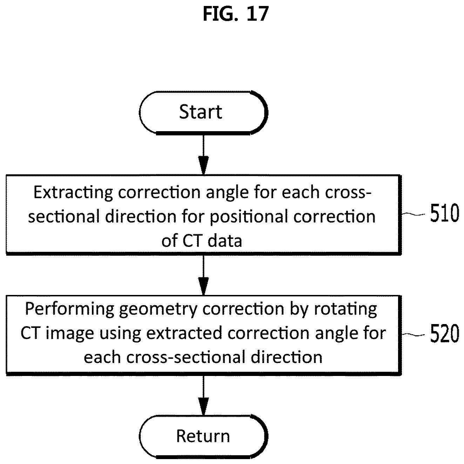

First, in step 500, the geometry correction unit 100 corrects so that a CT image is positioned at a correct position in a three-dimensional space by applying a CT geometry correction to CT data. The process of the geometry correction will be described later with reference to FIGS. 17 to 19.

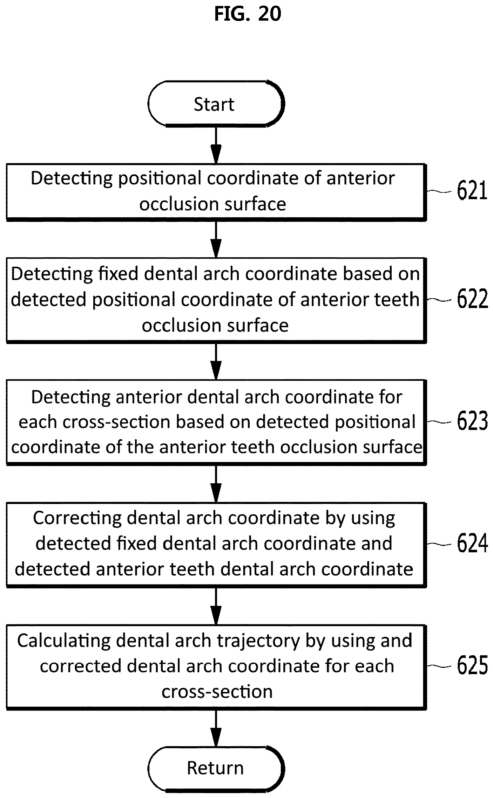

Then, in step 600, the dental arch trajectory detection unit 200 detects a dental arch trajectory. Herein, the step 600 of detecting of the dental arch trajectory includes: a step 610 of setting, by the position data setting part 210, a standard dental arch and an RCP coordinate by using device radiograph sequence information; a step 620 of generating, by the dental arch trajectory generator 220, a dental arch trajectory by using the standard dental arch set in the position data setting part 210. The step 620 of generating the dental arch trajectory will be described later with reference to FIG. 20.

Then, in step 700, the active section setting unit 300 sets an active image data section, and calculates an X-ray irradiation path for each position of the dental arch trajectory by setting a positional coordinate of an X-ray source for each position of the dental arch trajectory within the active image data section.

Next, in step 800, the reconstructing unit 400 performs a summation of "image data of the CT image corrected in the geometry correction unit 100" within "the active image data section set in the active section setting unit 300" for each X-ray irradiation path based on the dental arch trajectory detected in the dental arch trajectory detection unit 200, and reconstructs a panorama image by arranging the summation result according the dental arch trajectory. Herein, the step 800 of reconstructing the panorama image includes: a step 810 of generating, by the weight map generator 410, a weight map; a step 820 of adjusting, by the adjustor 420, a ratio of image reflected on a reconstructing panorama image; and a step 830 of reconstructing, by the medical image reconstructor 430, the panorama image by performing a summation of "image data within the active image data section set in the active section setting unit 300" and which is adjusted by the adjustor 420 according to a weight value of the weight map generator 410. In addition, the step 800 of reconstructing the panorama image further includes a step 840 of adjusting, by the image size adjustor 440, a size of the panorama image reconstructed in the medical image reconstructor 430. In addition, the step 800 of reconstructing the panorama image further includes a step 850 of performing, by the medical image processor 450, image processing to the panorama image reconstructed in the medical image reconstructor 430.

FIG. 17 is a detailed flowchart of the step 500 of the geometry correction according to the embodiment of the present invention.

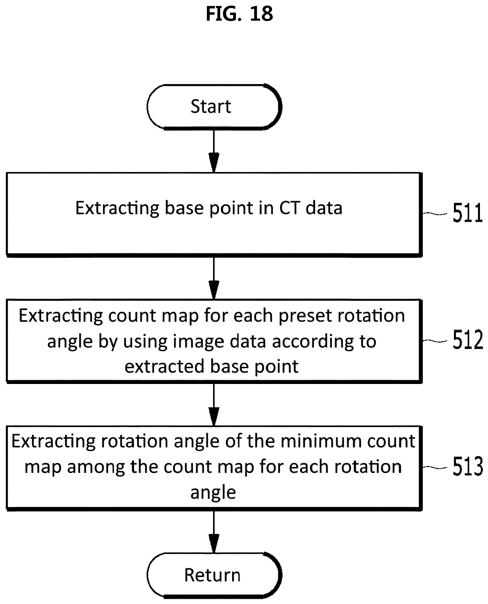

First, in step 510, the correction angle extractor 110 receives the CT data and extracts a correction angle for each cross-sectional direction for a positional correction of the CT data. Herein, in order to extract the correction angle for each cross-section, a sub-routine (correction angle extraction process of FIG. 18) may be performed for each cross-section.

Then, in step 520, the corrector 120 performs a geometry correction by rotating the CT image using the correction angle for each cross-sectional direction extracted in the correction angle extractor 110.

FIG. 18 is a detailed flowchart of the step 510 of extracting the correction angle according to the embodiment of the present invention.

First, in step 511, the base point extractor 111 extracts base point in the CT data.

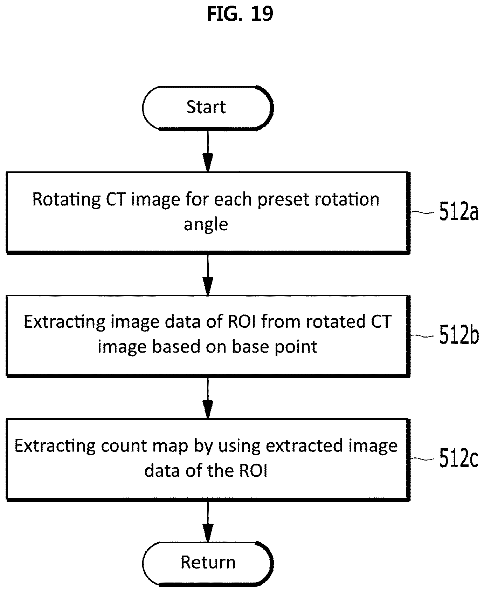

Then, in step 512, the rotation angle count map extractor 112 extracts a count map for each preset rotation angle by using image data according to the base point extracted in the base point extractor 111. Herein, in order to extract the count map for each rotation angle, a sub routine (rotation angle count map extraction process of FIG. 19) may be performed for each rotation angle.

Next, in step 513, the rotation angle extractor 113 extracts a rotation angle of the minimum count map among the count map for each rotation angle extracted in the rotation angle count map extractor 112.

FIG. 19 is a detailed flowchart of the step 512 of extracting the rotation angle count map according to the embodiment of the present invention.

First in step 512a, the image rotator 112a rotates the CT image for each preset rotation angle.

Then, in step 512b, the image data extractor 112b extracts image data of an ROI from the CT image rotated in the image rotator 112a based on the base point extracted in the base point extractor 111.

Next, in step 512c, the count map extractor 112c extracts a count map by using the image data of the ROI extracted in the image data extractor 112b.

FIG. 20 is a detailed flowchart of the step 620 of generating the dental arch trajectory according to the embodiment of the present invention.

First, in step 621, the positional coordinate detector 221 detects a positional coordinate of an anterior teeth occlusion surface.

Then, in step 622, the fixed dental arch coordinate detector 222 detects a fixed dental arch coordinate based on the positional coordinate of the anterior teeth occlusion surface detected in the positional coordinate detector 221.

Next, in step 623, the anterior dental arch coordinate detector 223 detects an anterior dental arch coordinate for each cross-section based on the positional coordinate of the anterior teeth occlusion surface detected in the positional coordinate detector 221.

Then, in step 624, the dental arch coordinate corrector 224 corrects a dental arch coordinate by using the fixed dental arch coordinate detected in the fixed dental arch coordinate detector 222 and the anterior dental arch coordinate detected in the anterior dental arch coordinate detector 223.

Next, in step 625, the dental arch trajectory calculator 225 calculates a dental arch trajectory by using the dental arch coordinate detected and corrected for each cross-section through the dental arch coordinate corrector 224.

Meanwhile, in the above embodiment, the CT image is corrected to be correctly positioned at the three-dimensional space by applying the CT geometry correction to the CT data.

Accordingly, when a CT image corrected according to the embodiment of the present invention is displayed through a viewer, regardless of a radiographing target and a posture, bilaterally symmetric organs such as eyes, ears, earlobes, etc., or feature points are permanently arranged on a horizontal surface.

A medical image reconstruction method in accordance with an example of the present invention may be implemented in the form of program instructions that can be executed by a variety of computer means, and may be stored in a computer-readable storage medium. The computer-readable storage medium may include program instructions, a data file, and a data structure solely or in combination. The program instructions that are stored in the medium may be designed and constructed particularly for the present invention, or may be known and available to those skilled in the field of computer software. Examples of the computer-readable storage medium include magnetic media such as a hard disk, a floppy disk and a magnetic tape, optical media such as CD-ROM and a DVD, magneto-optical media such as a floptical disk, and hardware devices particularly configured to store and execute program instructions such as ROM, RAM, and flash memory. Examples of the program instructions include not only machine language code that is constructed by a compiler but also high-level language code that can be executed by a computer using an interpreter or the like. The above-described hardware components may be configured to act as one or more software modules that perform the operation of the present invention, and vice versa.

While the present invention has been described in conjunction with specific details, such as specific configuration elements, and limited examples and diagrams above, these are provided merely to help an overall understanding of the present invention, the present invention is not limited to these examples, and various modifications and variations can be made from the above description by those having ordinary knowledge in the art to which the present invention pertains.

## Claims

1. A device for reconstructing a panoramic image of a dental arch using image data of a computed tomography, CT, image from a CT radiography device, comprising:

    a geometry correction unit (100) configured to correct a geometry of the image data so that the CT image is correctly positioned in a three-dimensional space;
    a dental arch trajectory detection unit (200) configured to detect a dental arch trajectory in the CT image;
    an active section setting unit configured to set an active image data section in the CT image; and
    a reconstructing unit (400) configured to generate a panoramic image by summating image data of the image data active section of the CT image corrected in the geometry correction unit (100),

**characterized in that**:

the dental arch trajectory detection unit (200) includes a position data setting part (210) configured to set a standard dental arch and a rotate center point ,RCP, coordinate , and a dental arch trajectory generator (220) configured to generate a dental arch trajectory by using the standard dental arch set in the position data setting part (210), wherein the RCP coordinate is calculated by using the detected dental arch trajectory and device radiograph sequence information,

the active section setting unit (300) is further configured to calculate an X-ray irradiation path of a panorama image radiograph sequence for each voxel position of the dental arch trajectory by setting a positional coordinate of an X-ray source for each voxel position of the dental arch trajectory within the active image data section based on the detected dental arch trajectory and the RCP coordinate, and

the reconstruction unit (400) is further configured to generate the panoramic image by performing a summation of the X-ray irradiation path generated from each positional coordinate and image data of intersection areas between dental arch trajectory and the X-ray irradiation path, and arranging the summation result according to the dental arch trajectory.

2. The device according to claim 1, the geometry correction unit (100) configured to extract angles of correction according to cross-sectional directions to correct a position of the CT image and perform geometry correction by rotating the CT image using the angles of correction according to cross-sectional directions, wherein the geometry correction unit (100) is configured to extract a base point information from the CT image, extract count maps according to preset angles of rotation using image information on basis of the base point information, and extract an angle of rotation of a minimum count map of the count maps extracted according to the angles of rotation.

3. The device according to claim 2, wherein the CT image is produced by obtaining a CT image of a head of a subject, and the base point information is one of position information about a jawline, information about an arrangement of teeth, position information about an ear plug, position information of a temporomandibular joint, TMJ, and position information of eyes in the CT image.

4. The device according to claim 2, wherein the geometry correction unit (100) is configured to rotate the CT image according to preset angles of rotation, extract image information of a region of interest on basis of the base point information from the rotated CT image, and extract the count maps using the image information of the region of interest.

5. The device according to claim 2, wherein the geometry correction unit (100) is configured to extract the angles of rotation of the minimum count map by measuring a number of valid pixels of the count maps extracted according to the angles of rotation and extracting a count map having a smallest number of pixels.

**Patentansprüche**

1. Vorrichtung zum Rekonstruieren eines Panoramabildes eines Zahnbogens unter Verwendung von Bilddaten eines Computertomographie-, CT-, Bildes von einem CT-Röntgengerät, umfassend:

eine Geometriekorrektureinheit (100), die so konfiguriert ist, dass sie eine Geometrie der Bilddaten korrigiert, so dass das CT-Bild in einem dreidimensionalen Raum korrekt positioniert ist;

eine Zahnbogen-Trajektorien-Erfassungseinheit (200), die so konfiguriert ist, dass sie eine Zahnbogen-Trajektorie in dem CT-Bild erfasst;

eine Einheit zum Einstellen eines aktiven Abschnitts, die so konfiguriert ist, dass sie einen aktiven Bilddatenabschnitt in dem CT-Bild einstellt; und

eine Rekonstruktionseinheit (400), die so konfiguriert ist, dass sie ein Panoramabild erzeugt, indem sie Bilddaten des aktiven Abschnitts der Bilddaten des CT-Bildes, die in der Geometriekorrektureinheit (100) korrigiert wurden, summiert,

**dadurch gekennzeichnet**:

die Zahnbogen-Trajektorien-Erfassungseinheit (200) ein Positionsdaten-Einstellteil (210), das so konfiguriert ist, dass es einen Standardzahnbogen und eine Drehmittelpunkt-, RCP-, Koordinate einstellt, und einen Zahnbogen-Trajektorien-Generator (220) umfasst, der so konfiguriert ist, dass er eine Zahnbogen-Trajektorie unter Verwendung des Standardzahnbogens, der in dem Positionsdaten-Einstellteil (210) eingestellt ist, erzeugt, wobei die RCP-Koordinate unter Verwendung der erfassten Zahnbogen-Trajektorie und der Röntgenbildsequenzinformation der Vorrichtung berechnet wird,

die aktive Abschnitts-Einstelleinheit (300) ferner so konfiguriert ist, dass sie einen Röntgenbestrahlungsweg einer Panoramabild-Röntgenbildsequenz für jede Voxelposition der Zahnbogenbahn berechnet, indem sie eine Positionskoordinate einer Röntgenquelle für jede Voxelposition der Zahnbogenbahn innerhalb des aktiven Bilddatenabschnitts auf der Grundlage der erfassten Zahnbogenbahn und der RCP-Koordinate einstellt, und

die Rekonstruktionseinheit (400) ferner so konfiguriert ist, dass sie das Panoramabild erzeugt, indem sie eine Summierung des Röntgenbestrahlungspfades, der von jeder Positionskoordinate und den Bilddaten der Schnittbereiche zwischen der Zahnbogenbahn und dem Röntgenbestrahlungspfad erzeugt wird, durchführt und das Summenergebnis entsprechend der Zahnbogenbahn anordnet.

2. Vorrichtung nach Anspruch 1, wobei die Geometriekorrektureinheit (100) so konfiguriert ist, dass sie Korrekturwinkel entsprechend den Querschnittsrichtungen extrahiert, um eine Position des CT-Bildes zu korrigieren und eine Geometriekorrektur durch Drehen des CT-Bildes unter Verwendung der Korrekturwinkel entsprechend den Querschnittsrichtungen durchzuführen,

wobei die Geometriekorrektureinheit (100) konfiguriert ist, um eine Basispunktinformation aus dem CT-Bild zu extrahieren, Zählkarten gemäß voreingestellten Drehwinkeln unter Verwendung von Bildinformation auf der Basis der Basispunktinformation zu extrahieren, und einen Drehwinkel einer minimalen Zählkarte der Zählkarten zu extrahieren, die gemäß den Drehwinkeln extrahiert wurden.

3. Vorrichtung nach Anspruch 2, wobei das CT-Bild durch Erhalten eines CT-Bildes eines Kopfes einer Person erzeugt wird und die Basispunktinformation eine der folgenden Informationen ist: Positionsinformation über eine Kieferlinie, Information über eine Anordnung von Zähnen, Positionsinformation über einen Ohrstöpsel, Positionsinformation eines Kiefergelenks, Kiefergelenk, und Positionsinformation von Augen im CT-Bild.

4. Vorrichtung nach Anspruch 2, wobei die Geometriekorrektureinheit (100) so konfiguriert ist, dass sie das CT-Bild gemäß voreingestellten Drehwinkeln dreht, Bildinformationen eines interessierenden Bereichs auf der Basis der Basispunktinformationen aus dem gedrehten CT-Bild extrahiert und die Zählkarten unter Verwendung der Bildinformationen des interessierenden Bereichs extrahiert.

5. Vorrichtung nach Anspruch 2, wobei die Geometriekorrektureinheit (100) so konfiguriert ist, dass sie die Drehwinkel der minimalen Zählkarte durch Messen einer Anzahl gültiger Pixel der Zählkarten, die gemäß den Drehwinkeln extrahiert wurden, und Extrahieren einer Zählkarte mit einer kleinsten Anzahl von Pixeln extrahiert.

**Revendications**

1. Dispositif pour reconstruire une image panoramique d'une arcade dentaire en utilisant les données d'image d'une tomographie assistée par ordinateur, CT, image provenant d'un appareil de radiographie CT, comprenant :

une unité de correction de la géométrie (100) configurée pour corriger une géométrie des données d'image afin que l'image CT soit correctement positionnée dans un espace tridimensionnel ;
une unité de détection de la trajectoire d'une arcade dentaire (200) configurée pour détecter une trajectoire d'arcade dentaire dans l'image CT ;
une unité de réglage de section active configurée pour régler une section de données d'image active dans l'image CT ; et
une unité de reconstruction (400) configurée pour générer une image panoramique en additionnant les données de la section active de l'image CT corrigée dans l'unité de correction de la géométrie (100),
caractérisé en cela :

l'unité de détection de trajectoire d'arc dentaire (200) comprend une partie de réglage de données de position (210) configurée pour définir une arcade dentaire standard et une coordonnée de point central de rotation, RCP, et un générateur de trajectoire d'arcade dentaire (220) configuré pour générer une trajectoire d'arcade dentaire en utilisant l'arcade dentaire standard définie dans la partie de réglage de données de position (210), dans laquelle la coordonnée RCP est calculée en utilisant la trajectoire d'arcade dentaire détectée et les informations de séquence radiographique du dispositif,

l'unité de réglage de la section active (300) est en outre configurée pour calculer un trajet d'irradiation par rayons X d'une séquence de radiographie d'image panoramique pour chaque position de voxel de la trajectoire de l'arcade dentaire en réglant une coordonnée de position d'une source de rayons X pour chaque position de voxel de la trajectoire de l'arcade dentaire dans la section de données d'image active sur la base de la trajectoire de l'arcade dentaire détectée et de la coordonnée RCP, et

l'unité de reconstruction (400) est en outre configurée pour générer l'image panoramique en effectuant une sommation du trajet d'irradiation des rayons X généré à partir de chaque coordonnée de position et des données d'image des zones d'intersection entre la trajectoire de l'arcade dentaire et le trajet d'irradiation des rayons X, et en arrangeant le résultat de la sommation en fonction de la trajectoire de l'arcade dentaire.

2. Le dispositif selon la revendication 1, l'unité de correction de la géométrie (100) configurée pour extraire des angles de correction selon les directions de la section transversale pour corriger une position de l'image CT et pour effectuer la correction de la géométrie en faisant tourner l'image CT en utilisant les angles de correction selon les directions de la section transversale,

dans lequel l'unité de correction de la géométrie (100) est configurée pour extraire une information de point de base de l'image CT, extraire des cartes de comptage selon des angles de rotation prédéfinis en utilisant l'information d'image sur la base de l'information de point de base, et extraire un angle de rotation d'une carte de comptage minimum des cartes de comptage extraites selon les angles de rotation.

3. Dispositif selon la revendication 2, dans lequel l'image CT est produite en obtenant une image CT d'une tête d'un sujet, et les informations de point de base sont des informations de position concernant un trait de mâchoire, des informations concernant un arrangement de dents, des informations de position concernant un bouchon d'oreille, des informations de position d'une articulation temporo-mandibulaire, l'ATM, et des informations de position des yeux dans l'image CT.

4. Dispositif selon la revendication 2, dans lequel l'unité de correction de la géométrie (100) est configurée pour faire tourner l'image CT selon des angles de rotation prédéfinis, extraire des informations d'image d'une région d'intérêt sur la base des informations de point de base de l'image CT tournée, et extraire les cartes de comptage en utilisant les informations d'image de la région d'intérêt.

5. Dispositif selon la revendication 2, dans lequel l'unité de correction de la géométrie (100) est configurée pour extraire les angles de rotation de la carte de comptage minimum en mesurant un nombre de pixels valides des cartes de comptage extraites selon les angles de rotation et en extrayant une carte de comptage ayant un nombre de pixels le plus petit.

## FIG. 1

CT Data

↓

| Geometry Correction Unit | ~ 100 |

↓

Dental Arch Trajectory Detector ⌐~ 200

| Position Data Setting Part | ~ 210 |

↓

| Dental Arch Trajectory Generator | ~ 220 |

↓

| Active Image Data Section | ~ 300 |

↓

Reconstructing Unit ⌐~ 400

| Weight Map Generator | ~ 410 |

↓

| Adjustor | ~ 420 |

↓

| Medical Image Reconstructor | ~ 430 |

↓

| Medical Image Size Adjustor | ~ 440 |

↓

| Medical Image Processor | ~ 450 |

↓

Reconstructed    Panorama    Image

# FIG. 2

CT Data

**Geometry Correction Unit** — 100

| Correction Angle Extractor | — 110 |

| Corrector | — 120 |

Geometrically Corrected Medical Image Data

# FIG. 3

CT Data — 110

**Correction Angle Extractor**

| Base Point Extractor | — 111 |

| Rotation Angle Count Map Extractor | — 112 |

| Rotation Angle Extractor | — 113 |

Rotation Angle (Correction Angle)

# FIG. 4

Base Point
112

Rotation Angle Count Map
Extractor

| Image Rotator | 112a |
|---|---|

| Image Data Extractor | 112b |
|---|---|

| Count Map Extractor | 112c |
|---|---|

Count Map

# FIG. 5

| Positional Coordinate Detector | 221 |
|---|---|

| Fixed Dental Arch Coordinate Detector | 222 |
|---|---|

| Anterior Dental Arch Coordinate Detector | 223 |
|---|---|

| Dental Arch Coordinate Corrector | 224 |
|---|---|

| Dental Arch Trajectory Calculator | 225 |
|---|---|

## FIG. 6

## FIG. 7

## FIG. 8

## FIG. 9

## FIG. 10

Anterior Teeth Occlusal Surface

135°

## FIG. 11

Min Cut    Dental Arch Coordinate    Max Cut

## FIG. 12

## FIG. 13

Min Cut      Dental Arch Coordinate      Max Cut

## FIG. 14

## FIG. 15

Min Cut   Dental Arch Coordinates    Max Cut

# FIG. 16

Start

Correcting CT image to be positioned at correct position in three-dimensional space by applying CT geometry correction — 500

Detecting dental arch trajectory — 600

Setting standard dental arch and RCP coordinate by using radiograph sequence information of device — 610

Generating dental arch trajectory by using standard dental arch — 620

Setting active image data section, and calculating X-ray irradiation path for each position of dental arch trajectory by setting positional coordinate of X-ray source for each position of dental arch trajectory within image data active section — 700

Performing summation of "image data of the CT image" within " active image data section" for each X-ray irradiation path based on detected dental arch trajectory, and reconstructing panorama image by arranging summation result according to dental arch trajectory — 800

Generating weighting map — 810

Adjusting ratio of image data reflected on reconstructing Panorama image — 820

Reconstructing Panorama image by performing summation of "image data within active image data section" and which is adjusted according to weight value — 830

Adjusting size of reconstructed panorama image — 840

Performing image processing to reconstructed panorama image — 850

End

**FIG. 17**

```
        ┌─────────────┐
        │    Start    │
        └──────┬──────┘
               │
               ▼
┌──────────────────────────────────┐
│  Extracting correction angle for  │
│  each cross-sectional direction   │──── 510
│  for positional correction        │
│  of CT data                       │
└──────────────┬───────────────────┘
               │
               ▼
┌──────────────────────────────────┐
│  Performing geometry correction   │
│  by rotating CT image using       │──── 520
│  extracted correction angle for   │
│  each cross-sectional direction   │
└──────────────┬───────────────────┘
               │
               ▼
        ┌─────────────┐
        │   Return    │
        └─────────────┘
```

**FIG. 18**

```
        ┌──────────┐
        │  Start   │
        └────┬─────┘
             │
             ▼
┌────────────────────────────────┐
│  Extracting base point in CT data  │ ─── 511
└────────────────┬───────────────┘
                 │
                 ▼
┌────────────────────────────────┐
│ Extracting count map for each preset rotation │
│  angle by using image data according to       │ ─── 512
│          extracted base point                 │
└────────────────┬───────────────┘
                 │
                 ▼
┌────────────────────────────────┐
│ Extracting rotation angle of the minimum count │
│  map among the count map for each rotation     │ ─── 513
│                 angle                          │
└────────────────┬───────────────┘
                 │
                 ▼
        ┌──────────┐
        │  Return  │
        └──────────┘
```

**FIG. 19**

```
        ╭─────────────╮
        │    Start     │
        ╰──────┬──────╯
               │
               ▼
  ┌────────────────────────────┐
  │ Rotating CT image for each  │ ── 512a
  │  preset rotation angle      │
  └──────────────┬─────────────┘
               │
               ▼
  ┌────────────────────────────┐
  │ Extracting image data of ROI│ ── 512b
  │ from rotated CT image based │
  │        on base point        │
  └──────────────┬─────────────┘
               │
               ▼
  ┌────────────────────────────┐
  │ Extracting count map by using│ ── 512c
  │  extracted image data of the │
  │            ROI               │
  └──────────────┬─────────────┘
               │
               ▼
        ╭─────────────╮
        │   Return     │
        ╰─────────────╯
```

## FIG. 20

Start

Detecting positional coordinate of anterior occlusion surface — 621

Detecting fixed dental arch coordinate based on detected positional coordinate of anterior teeth occlusion surface — 622

Detecting anterior dental arch coordinate for each cross-section based on detected positional coordinate of the anterior teeth occlusion surface — 623

Correcting dental arch coordinate by using detected fixed dental arch coordinate and detected anterior teeth dental arch coordinate — 624

Calculating dental arch trajectory by using and corrected dental arch coordinate for each cross-section — 625

Return

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020130138612
- US 2011026671 A1

- US 2009052617 A1

### Non-patent literature cited in the description

- Develoment of imaging selction criteria and procedures should precede cephalometric assessment with cone-beam computed tomography. **FARMAN et al.** American Journal of Orthodontics and dentofacial orthope. Mosby, 01 August 2006, 257-265